# EUROPEAN PATENT APPLICATION

(11) **EP 2 724 703 A1**
(43) Date of publication of application: **30.04.2014**
(21) Application number: 12803107.7
(22) Date of filing: 08.06.2012
(51) Int. Cl.: A61J 3/00

(54) **CELL CRYOPRESERVATION CONTAINER**

(30) Priority: 22.06.2011 JP 2011138403
(71) Applicant: JMS Co., Ltd., Hiroshima-shi, Hiroshima 730-8652 (JP)
(72) Inventor: HIRAI, Satoshi, Hiroshima-shi Hiroshima 730-8652 (JP)
(74) Representative: Hirsch & Associés
(86) International application number: PCT/JP2012/064776
(87) International publication number: WO 2012/176630

(57) **Abstract**

Provided is a cell cryopreservation container which is provided with a plurality of storage chambers and enables efficient storage of cells in a definite capacity. The cell cryopreservation container (1) comprises a plurality of storage chambers (2) which are located in a first direction (WD) at definite intervals, dividing areas (3) which are located between two adjacent storage chambers (2), and communicating parts (4) which are located at one end side and/or the other end side in a second direction (HD) of the dividing area (3) and connect the two storage chambers (2), wherein a front face part (11) is formed in one side in a third direction (TD) while a back face part (12) is formed in the other side in the third direction (TD). In the cell cryopreservation container (1), the front face part (11) has a convex and concave shape configured of the storage chambers (2) projecting toward one side in the third direction (TD) while the back face part (12) has a planar shape, and the length in the first direction (WD) of the dividing areas (3) exceeds the length in the first direction (WD) of the storage chambers (2).

## Description

### TECHNICAL FIELD

The present invention relates to a cell cryopreservation container for preserving, for example, stem cells that are collected from living specimens.

### BACKGROUND ART

In recent years, stem cells collected from living specimens are used in the field of regeneration medicine for various purposes, such as hematopoietic stem cell transplantations and reconstruction of various organizations. After being collected from a living body and until prior to being used for regeneration medicine, stem cells are cryopreserved in a container filled with liquid nitrogen in a state where the stem cells are stored in the cell cryopreservation container (for example, refer to Patent Document 1).

A cell cryopreservation container proposed in Patent Document 1 includes: a first storage chamber which is constituted by flexible resin members and can store relatively large amount of cells (for example, 20 ml); a second storage chamber which can store relatively small amount of cells (for example, 5 ml); a dividing area which divides the first storage chamber and the second storage chamber; and a communicating part which is provided in the dividing area and connects the first storage chamber and the second storage chamber.

According to the cell cryopreservation container proposed in Patent Document 1, the first storage chamber and the second storage chamber can be isolated from each other by welding or the like the communicating part after storing the cells in the first storage chamber and the second storage chamber. Accordingly, the cells can be used by unfreezing only the first storage chamber or the second storage chamber, and thus the cryopreserved cells can be used efficiently according to the required amount of cells.

In addition, there has been proposed a cell cryopreservation container that has three or more storage chambers that can store a relatively small amount of cells in order to use cryopreserved cells multiple times by unfreezing a small amount each time.

[Patent Document 1] Japanese Unexamined Patent Application (Translation of PCT Application), Publication No. 2001-517103

### DISCLOSURE OF THE INVENTION

### Problems to be Solved by the Invention

Cell cryopreservation containers such as the above are cryopreserved in a state where the cell cryopreservation containers are stored in a protective case constituted by metal materials, such as stainless steel and aluminum. Here, when the number of storage chambers is increased in a cell cryopreservation container, the number of the dividing areas that divide adjacent storage chambers increases also, and therefore the proportion of the area of the dividing areas to the cell cryopreservation container increases. Therefore, when a cell cryopreservation container that includes many storage chambers is stored in a protective case, there are produced unnecessary spaces inside the protective case where cells are not stored. As a result, there is a problem that cells cannot be efficiently stored in liquid nitrogen since the amount of cells that can be stored in a protective case having the same capacity is reduced in a cell cryopreservation container that includes many storage chambers as compared to a cell cryopreservation container that has small number of storage chambers.

Therefore, an object of the present invention is to provide a cell cryopreservation container that has a plurality of storage chambers and enables efficient storage of cells in a definite capacity.

### Means for Solving the Problems

The present invention relates to a cell cryopreservation container that includes: a plurality of storage chambers which are arranged at definite intervals in a first direction; one or a plurality of dividing areas which are arranged between two adjacent storage chambers; and one or a plurality of communicating parts which are arranged at one end side and/or the other end side in the dividing area in a second direction that is orthogonal to the first direction and connect the two adjacent storage chambers; wherein a front face part is formed at one side in a third direction that is orthogonal to the first direction and the second direction and a back face part is formed at the other side in the third direction; the front face part is formed in a convex and concave shape by the plurality of storage chambers and the communicating part protruding to the one side in the third direction and the back face part is formed planate; and length of the dividing area in the first direction is longer than length of the storage chamber in the first direction.

In addition, it is preferable if an end surface in each of the plurality of storage chambers at the front face part side and a surface of the dividing area at the front face part side are formed planate.

In addition, it is preferable if the communicating part includes: a first communicating part which is arranged in the dividing area at a side of one end in the second direction; and a second communicating part which is arranged in the dividing area at a side of the other end in the second direction.

In addition, it is preferable if the first communicating part is arranged at the one end side in the second direction as compared to a position of one end part of the storage chamber in the second direction, and the second communicating part is arranged at the other end side in the second direction as compared to a position of the other end part of the storage chamber in the second direction.

In addition, it is preferable if length of the communicating part in the third direction is less than or equal to 1/2 of the length of the storage chamber in the third direction.

In addition, it is preferable if the cell cryopreservation container further comprises: a cell introduction part which is provided in one of the plurality of storage chambers and via which a cell is introduced into the plurality of storage chambers; and a cell withdrawal part which is provided in the plurality of storage chambers and via which the cell stored in the storage chamber is withdrawn.

In addition, it is preferable if the cell withdrawal part is provided at the one end part of each of the plurality of storage chambers in the second direction, and the cell introduction part is provided at the other end part of one storage chamber in the second direction out of the plurality of storage chambers.

In addition, it is preferable if the dividing area includes a first dividing area whose length in the first direction is a first length and a second dividing area whose length in the first direction is a second length, which is longer than the first length, and the second dividing area is arranged between two of the storage chambers located in a center out of the plurality of storage chambers.

In addition, it is preferable if the communicating part is not arranged in the second dividing area.

### Effects of the Invention

According to the cell cryopreservation container of the present invention, it is possible to have a plurality of storage chambers and efficiently store the cells in a definite capacity.

### BRIEF DESCRIPTION OF THE DRAWINGS

FIG. 1 is a perspective view showing a cell cryopreservation container according to a first embodiment of the present invention;
FIG. 2 is a plan view showing the cell cryopreservation container according to the first embodiment;
FIG. 3 is a cross-sectional view of Line A-A in FIG. 2;
FIG. 4 is a cross-sectional view of Line B-B line in FIG. 2;
FIG. 5 is a cross-sectional view of Line C-C line in FIG. 2;
FIG. 6 is a view from Arrow D in FIG. 2;
FIG. 7 is a perspective view showing a cylindrical member;
FIG. 8A is a perspective view showing a protective case for storing the cell cryopreservation container;
FIG. 8B is a perspective view showing a state where a first cell cryopreservation container is stored in the protective case;
FIG. 8C is a perspective view showing a state where a second cell cryopreservation container is stored in the protective case;
FIG. 8D is a perspective view showing a state where a lid part of the protective case is closed;
FIG. 8E is a cross-sectional view of D-D line in FIG. 8D;
FIG. 9A is a schematic view showing a cubic figure formation process in a method for manufacturing a cell cryopreservation container according to the first embodiment;
FIG. 9B is a schematic view showing a lamination arrangement process in the method for manufacturing a cell cryopreservation container according to the first embodiment;
FIG. 9C is a schematic view showing a first welding process in the method for manufacturing a cell cryopreservation container according to the first embodiment;
FIG. 9D is a schematic view showing a second welding process in the method for manufacturing a cell cryopreservation container according to the first embodiment;
FIG. 10 is a plan view showing a cell cryopreservation container according to a second embodiment;
FIG. 11 is a plan view showing a cell cryopreservation container according to a third embodiment;
FIG. 12 is a view from Arrow F in FIG. 11;
FIG. 13 is a side view showing a state where the cell cryopreservation container according to the third embodiment is folded;
FIG. 14 is a plan view showing a modification example of the cell cryopreservation container according to the third embodiment;
FIG. 15 is a plan view showing a first modification example of a communicating part of the cell cryopreservation container; and
FIG. 16 is a plan view showing a second modification example of the communicating part of the cell cryopreservation container.

### EXPLANATION OF REFERENCE NUMERALS

1: cell cryopreservation container
2: storage chamber
3: dividing area
4: communicating part
41: first communicating part
42: second communicating part
6: cell introduction part
7: cell withdrawal part
11: front face part
12: back face part
WD: width direction (first direction)
HD: height direction (second direction)
TD: thickness direction (third direction)

### PREFERRED MODE FOR CARRYING OUT THE INVENTION

Hereafter, preferred embodiments of the cell cryopreservation container according to the present invention will be described with reference to the drawings. The cell cryopreservation container 1 of the present invention is used when cryopreserving cells, such as stem cells collected from living specimens.

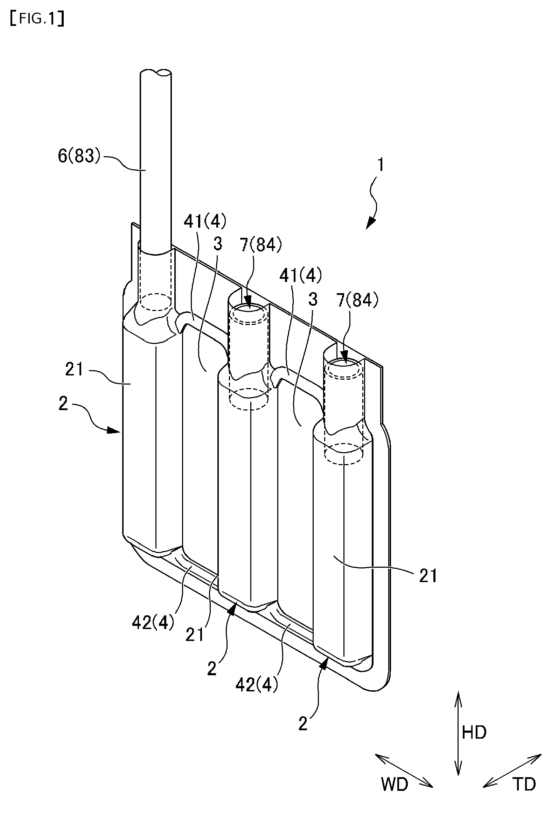

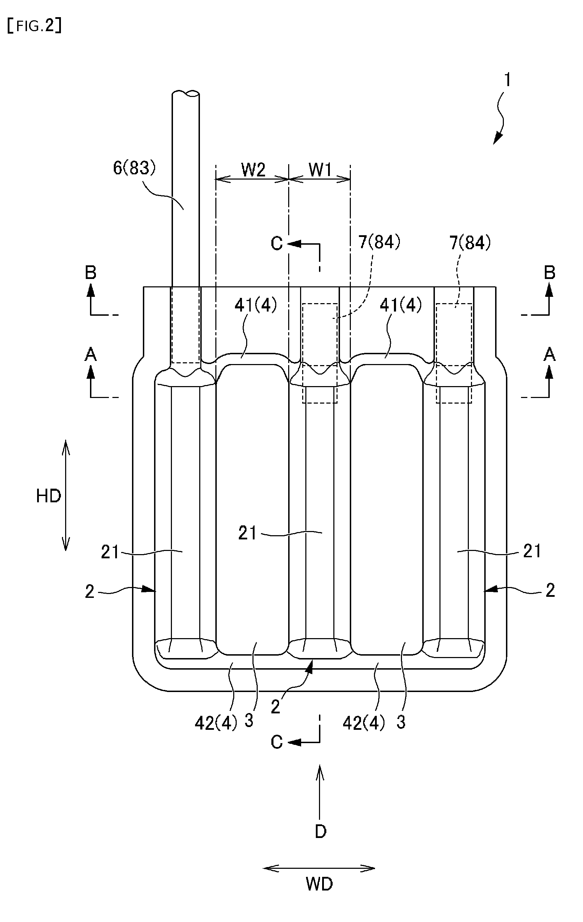

First, the cell cryopreservation container 1 according to the first embodiment will be described with reference to FIG. 1 to FIG. 7. As shown in FIG. 1 and FIG. 2, the cell cryopreservation container according to the first embodiment has predetermined width, height and thickness and is formed in a rectangular shape in a planar view. As shown in FIG. 1 and FIG. 2, the cell cryopreservation container 1 includes: a plurality of storage chambers 2; a dividing area 3 which divides the plurality of storage chambers 2; a communicating part 4 which connects two adjacent storage chambers 2; a cell introduction part 6 via which cells are introduced into the storage chamber 2; and a cell withdrawal part 7 via which the cells stored in the storage chamber 2 are withdrawn.

It should be noted that the present invention will be described hereinafter by using examples where a width direction WD of the cell cryopreservation container 1 is a first direction, a height direction HD of the cell cryopreservation container 1 is a second direction that is orthogonal to the first direction, and a thickness direction TD of the cell cryopreservation container 1 is a third direction that is orthogonal to the first direction and the second direction.

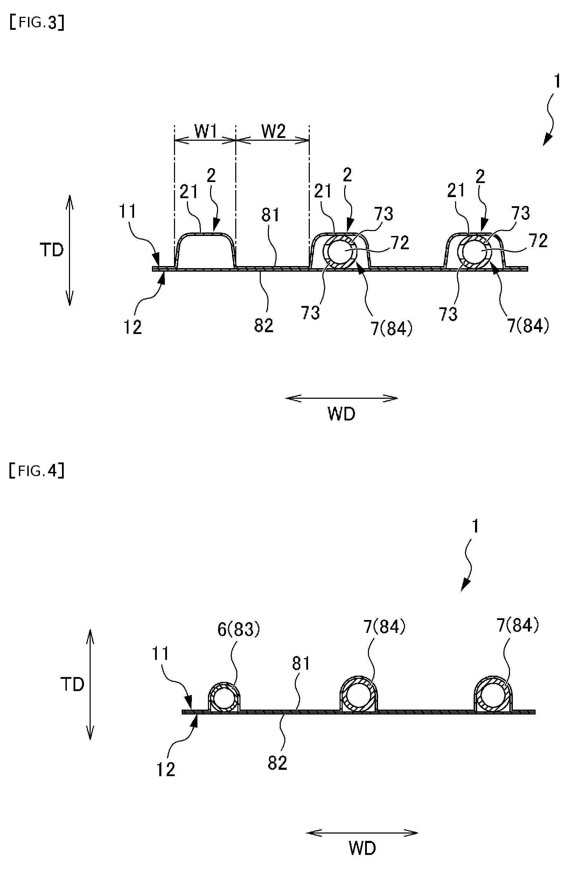

As shown in FIG. 1 and FIG. 2, a plurality of storage chambers 2 are arranged at definite intervals in the width direction WD. As shown in FIG. 1 to FIG. 3, the plurality of storage chambers 2 are configured in a substantially rectangular parallelepiped shape in which length (height) in the height direction HD is longer than length (width) in the width direction WD and length (thickness) in the thickness direction TD. More specifically, as shown in FIG. 3, the plurality of storage chambers 2 are formed so as to protrude onto one side in the thickness direction TD. Moreover, the protruding end face 21, which is an end face of the protruding side, of each of the plurality of storage chambers 2 is configured planate. In addition, the surface of the plurality of storage chambers 2 at the other side in the thickness direction TD is formed planate.

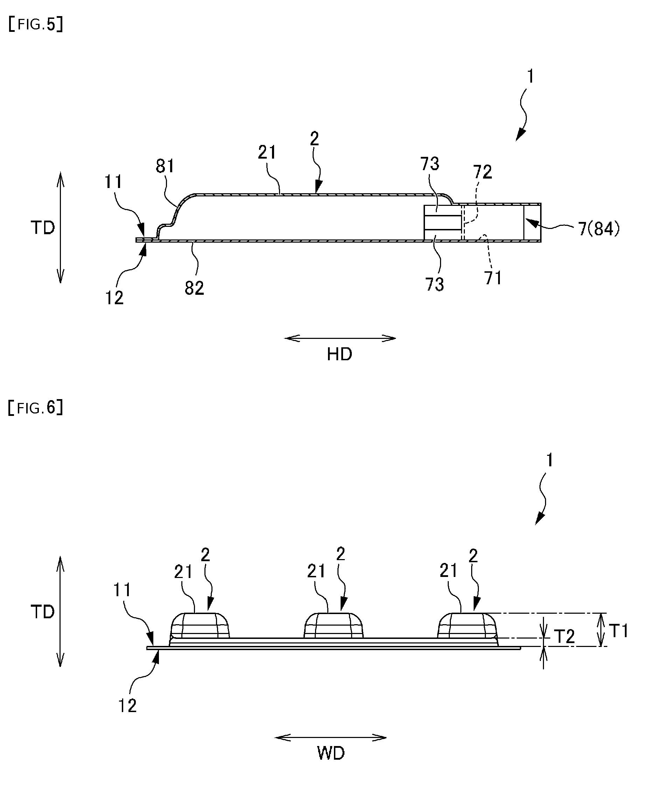

As shown in FIG. 3 and FIG. 5, both the sectional shape of the plurality of storage chambers 2 in the width direction WD, and the cross section of the plurality of storage chambers 2 in the height direction HD are formed in a trapezoidal shape in which the width becomes narrow gradually toward the protruding end face 21.

Cells collected from living specimens are stored in the plurality of storage chambers 2. In the first embodiment, three storage chambers 2 are arranged at definite intervals in the width direction WD. Capacity of each of the plurality of storage chambers 2 is preferably 1 ml to 10 ml from the viewpoint of unfreezing the cells efficiently in cases where the amount for one use is relatively small.

The dividing area 3 is arranged between two adjacent storage chambers 2. In the first embodiment, as shown in FIG. 1 and FIG. 2, two dividing areas 3 are formed. It is configured such that the length W2 of the dividing area 3 in the width direction WD is longer than the length W1 of the storage chamber 2 in the width direction WD. More specifically, the length W2 of the dividing area 3 in the width direction WD is preferably 100% to 150% of the length W1 of the storage chamber 2 in the width direction WD.

The communicating part 4 includes a plurality of first communicating parts 41 and a plurality of second communicating parts 42 as shown in FIG. 1 and FIG. 2.

As shown in FIG. 2, the first communicating part 41 is arranged at the upper end side, which is one end side in the height direction HD, of each of the plurality of (two) dividing areas 3. The first communicating part 41 connects the two adjacent storage chambers 2.

The first communicating part 41 is arranged at the upper end side as compared to the upper end part of the storage chamber 2 as shown in FIG. 2. In the first embodiment, the first communicating part 41 is arranged at the upper end side as compared to the position of the upper end part of the protruding end face 21 of the storage chamber 2.

As shown in FIG. 2, the second communicating part 42 is arranged at the lower end side, which is the other end side in the height direction HD, of each of the plurality of (two) dividing areas 3. The second communicating part 42 connects the two adjacent storage chambers 2.

As shown in FIG. 2, the second communicating part 42 is arranged at the lower end side as compared to the lower end part of the storage chamber 2. In the first embodiment, the second communicating part 42 is arranged at the lower end side as compared to the position of the lower end part of the protruding end face 21 of the storage chamber 2.

As shown in FIG. 6, the above first communicating part 41 and second communicating part 42 are formed to protrude onto one side in the thickness direction TD.

The thickness (length in the thickness direction TD) T2 of the first communicating part 41 and the second communicating part 42 is preferably less than or equal to 1/2, and more preferably less than or equal to 1/4, of thickness (length in the thickness direction TD) T1 of the storage chamber 2.

The cell introduction part 6 is formed at one storage chamber 2 out of the plurality of storage chambers 2 and serves as an introduction port for the cells in a case of introducing the cells into the plurality of storage chambers 2. In the first embodiment, the cell introduction part 6 is provided at a storage chamber 2 located on one end part in the width direction WD (left end shown in FIG. 2) out of the three storage chambers 2. In addition, the cell introduction part 6 is arranged at the upper end part in the storage chamber 2.

In the first embodiment, the cell introduction part 6 is constituted by a tube 83 made of a thermoplastic resin such as EVA resin (ethylene-vinyl acetate copolymer resin), and one end part of the tube 83 is connected to the upper end part of the storage chamber 2 and the other end part of the tube 83 extends upward.

The cell withdrawal part 7 serves as a withdrawal port in the case of withdrawing the cells stored in the plurality of storage chambers 2. The cell withdrawal part 7 is formed at the storage chamber 2 where the cell introduction part 6 is not formed out of the plurality of storage chambers 2 as shown in FIG. 1 and FIG. 2. In addition, the cell withdrawal part 7 is arranged at the upper end part in the storage chamber 2.

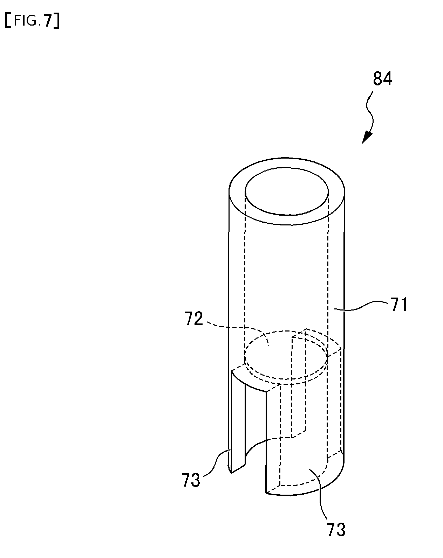

In the first embodiment, the cell withdrawal part 7 is constituted by a cylindrical member 84 made of thermoplastic resin such as EVA resin. More specifically, as shown in FIG. 7, the cylindrical member 84 includes: a cylindrical part 71; a closing film part 72 which closes one end side of the cylindrical part 71 in the longitudinal direction; and a pair of guard parts 73 which extend in the longitudinal direction from the one end side of the cylindrical part 71.

As shown in FIG. 3, the cylindrical member 84 is arranged such that the pair of guard parts 73 are located inside the storage chamber 2 and the pair of guard parts 73 are located at one side (the side of the front face part 11 described later) and the other side (the side of the back face part 12 described later) in the thickness direction TD.

As shown in FIG. 3 to FIG. 6, in the above cell cryopreservation container 1, the front face part 11 having a convex and concave shape is formed at one side in the thickness direction TD and the back face part 12 of a planar shape is formed at the other side in the thickness direction TD.

More specifically, as described above, the plurality of storage chambers 2, the first communicating part 41, the second communicating part 42, the cell introduction part 6, and the cell withdrawal part 7 protrude to one side in the thickness direction TD (the side of the surface part 11), and thus there is formed a convex-shaped part in the front face part 11 with the plurality of storage chambers 2, the first communicating part 41, and the second communicating part 42. In addition, the side of the front face part 11 of the dividing area 3 is formed planate and there is formed a concave-shaped part in the front face part 11 by the region where the dividing area 3 is located.

In addition, neither of the plurality of storage chambers 2, the dividing area 3, the first communicating part 41, the second communicating part 42, the cell introduction part 6, nor the cell withdrawal part 7 protrudes to the side of the back face part 12, thus the back face part 12 is formed planate.

The cell cryopreservation container 1 according to the first embodiment is used as follows.

When introducing cells into the cell cryopreservation container 1 according to the first embodiment, a syringe (not illustrated) or the like in which the cells collected from living specimens are stored is first connected to the tube 83 constituting the cell introduction part 6. Subsequently, the cells stored in the syringe or the like are introduced into the storage chamber 2 provided with the cell introduction part 6. Here, the lower end side of the plurality of storage chambers 2 is connected by the second communicating part 42, and the upper end part of the plurality of storage chambers 2 is connected by the first communicating part 41. Therefore, the cells introduced into the storage chamber 2 provided with the cell introduction part 6 are introduced into all of the plurality of storage chambers 2 mainly through the second communicating part 42. In addition, air inside the plurality of storage chambers 2 moves into the storage chamber 2 provided with the cell introduction part 6 mainly through the first communicating part 41 and is thereafter led out to the syringe or the like from the cell introduction part 6. Thereby, the cells are stored in the plurality of storage chambers 2.

Subsequently, the tube 83 of the cell cryopreservation container 1 in which the cells are stored is cut off by being melted and thus the cell introduction part 6 is hermetically-sealed. In addition, if necessary, the first communicating part 41 and the second communicating part 42 are blocked by welding or the like, thus separating the plurality of storage chambers 2 with each other.

In this state, the cell cryopreservation container 1 is cryopreserved.

When using the cryopreserved cells stored in the cell cryopreservation container 1, the storage chamber(s) 2 of the number corresponding to the necessary amount is unfrozen by separating at a part where the first communicating part 41 and the second communicating part 42 have been welded. Subsequently, the unfrozen cells stored in the storage chamber 2 are collected from the cell withdrawal part 7 using, for example, a syringe (not illustrated) on which a syringe needle is installed at the tip. More specifically, when collecting the cells from the cell withdrawal part 7, the syringe needle installed on the syringe is led into the closing film part 72 of the cylindrical member 84 so as to penetrate the closing film part 72, thus collecting the cells stored inside the storage chamber 2.

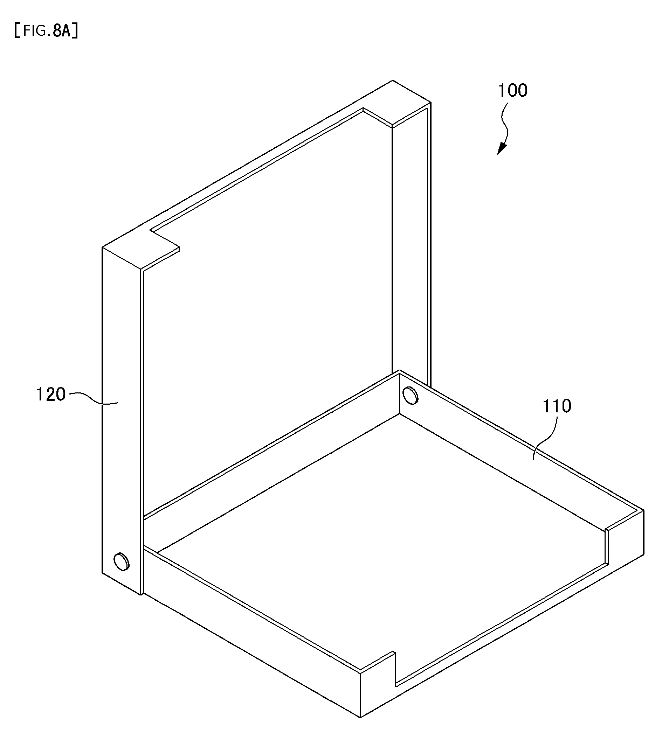

Next, the method for cryopreserving the cell cryopreservation container 1 that stores cells will be described with reference to FIG. 8A to FIG. 8E. FIG. 8A to FIG. 8E are respectively drawings showing a situation where the cell cryopreservation container 1 is cryopreserved.

As shown in FIG. 8A, the cell cryopreservation container 1 where the cells are introduced into the plurality of storage chambers 2 from the cell introduction part 6 is cryopreserved in liquid nitrogen in a state where the cell cryopreservation container 1 is stored in a protective case 100 which includes a main body part 110 of which the top face is opened, and a lid part 120 which is attached to the main body part 110 freely openable and closable and can cover the top face of the main body part 110.

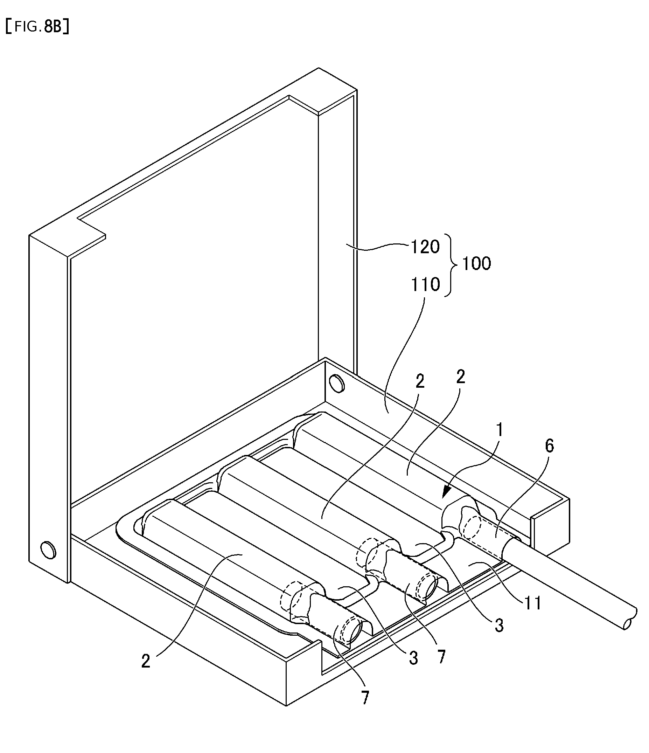

Here, when preserving the cell cryopreservation container 1 in the protective case 100, the first cell cryopreservation container 1 is first stored in the main body part 110. Here, as shown in FIG. 8B, the first cell cryopreservation container 1 is stored in the main body part 110 such that the back face part 12 is oriented toward the bottom side of the main body part 110. As a result, the side of the front face part 11 of the first cell cryopreservation container 1 is arranged at the top face side of the main body part 110.

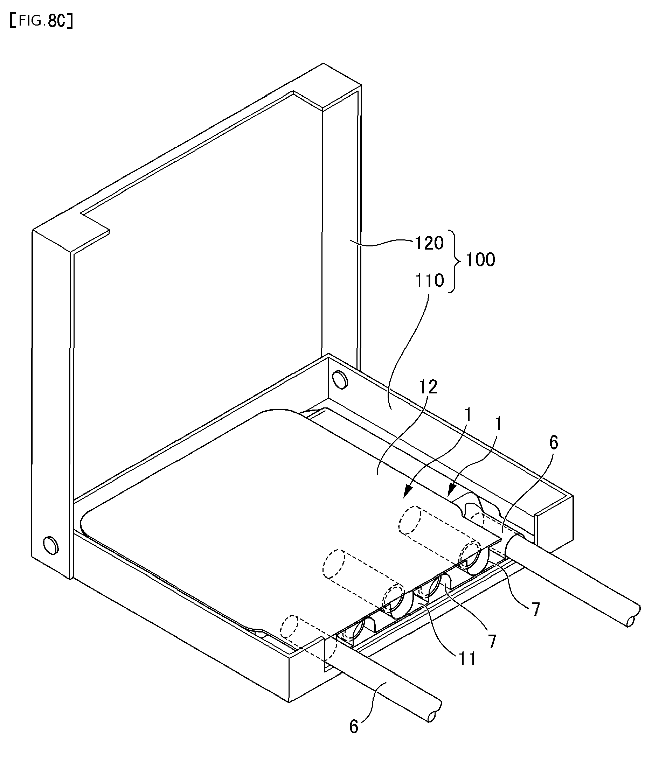

Subsequently, the second cell cryopreservation container 1 is arranged so as to overlap with the first cell cryopreservation container 1 stored in the main body part 110. At this time, as shown in FIG. 8C, the second cell cryopreservation container 1 is arranged such that the front face part 11 opposes against the front face part 11 of the first cell cryopreservation container 1. Here, the convex-shaped part formed by the storage chamber 2 and the concave-shaped part formed by the dividing area 3 are arranged alternately on the front face part 11 of the cell cryopreservation container 1. In addition, length W2 of the dividing area 3 in the width direction WD is configured to be wider than length W1 of the storage chamber 2 in the width direction WD. Furthermore, the back face part 12 of the cell cryopreservation container 1 is formed planate. Accordingly, by way of arranging the second cell cryopreservation container 1 overlapped with the first cell cryopreservation container 1 such that the storage chamber 2 of the second cell cryopreservation container 1 and the dividing area 3 of the first cell cryopreservation container 1 oppose each other, it is possible to store the second cell cryopreservation container 1 using the space formed with the dividing area 3 (concave-shaped part) in a state where the first cell cryopreservation container 1 is stored. Therefore, it is possible to store the two cell cryopreservation containers 1 without forming unnecessary space in the main body part 110.

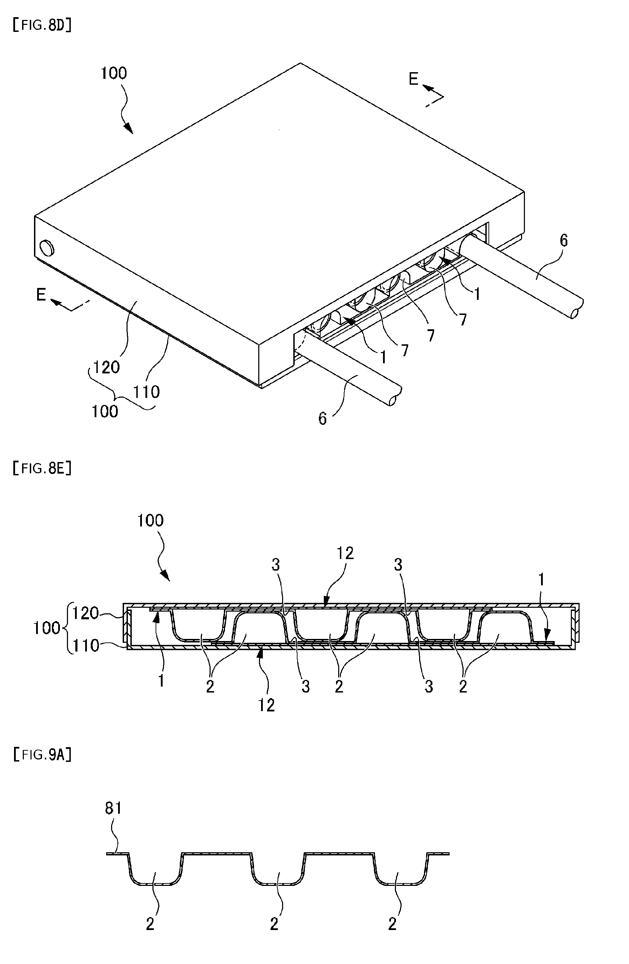

Subsequently, as shown in FIG. 8D, the top face of the main body part 110 is covered by closing the lid part 120. Thereby, the first cell cryopreservation container 1 and the second cell cryopreservation container 1 are stored in the protective case 100. Here, since the back face part 12 of the cell cryopreservation container 1 is formed planate, unnecessary space is not formed between the bottom surface of the main body part 110 and the back face part 12 of the first cell cryopreservation container 1 and between the back face part 12 of the second cell cryopreservation container 1 and the inner surface of the lid part 120 in a state where two cell cryopreservation containers 1 are stored in the protective case 100 (refer to FIG. 8E).

Next, the method for manufacturing a cell cryopreservation container 1 according to the first embodiment will be described with reference to FIG. 9A to FIG. 9D. FIG. 9A to FIG. 9D are drawings schematically showing the manufacturing processes of the cell cryopreservation container 1 according to the first embodiment.

As shown in FIG. 3 to FIG. 5, the cell cryopreservation container 1 according to the first embodiment is constituted by: a first sheet-shaped member 81 that constitutes the side of the front face part 11; a second sheet-shaped member 82 that constitutes the side of the back face part 12; a tube 83 which constitutes the cell introduction part 6; and a cylindrical member 84 which constitutes the cell withdrawal part 7. All of the first sheet-shaped member 81, the second sheet-shaped member 82, the tube 83, and the cylindrical member 84 are constituted by EVA resin.

The method for manufacturing a cell cryopreservation container 1 includes a cubic figure formation process S1, a lamination arrangement process S2, a first welding process S3, and a second welding process S4.

As shown in FIG. 9A, in the cubic figure formation process S1, a shaping operation is performed onto the first sheet-shaped member 81 and a cubic figure corresponding to the shape of the plurality of storage chambers 2, the first communicating part 41, and the second communicating part 42 (not illustrated) is formed on the first sheet-shaped member 81. The cubic figure formation process S1 is carried out with a vacuum shaping technique which brings, for example, the heated first sheet-shaped member 81 close contact with the surface of a mold (not illustrated) having a shape corresponding to the shape of the plurality of storage chambers 2, the first communicating part 41, and the second communicating part 42, and then sucks from the rear face side of the mold the first sheet-shaped member 81 thus brought into close contact with the surface of the mold.

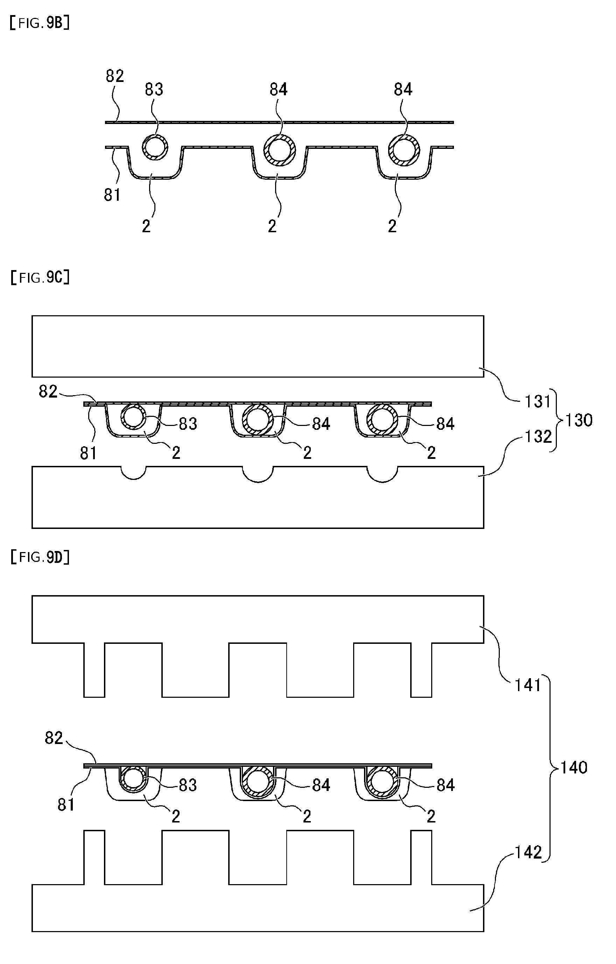

In the lamination arrangement process S2, as shown in FIG. 9B, the tube 83 and the cylindrical member 84 are arranged on the top face of the first sheet-shaped member 81 that is arranged such that the outer surface side (the side that has become convex in cubic figure) serves as the undersurface, and the second sheet-shaped member 82 is further laminated thereon.

In the first welding process S3, as shown in FIG. 9C, by using a first welding mold 130, which is constituted by an upper mold 131 and a lower mold 132, the tube 83 and the cylindrical member 84, which are arranged so as to be sandwiched between the first sheet-shaped member 81 and the second sheet-shaped member 82, are welded with the first sheet-shaped member 81 and the second sheet-shaped member 82.

In the second welding process S4, as shown in FIG. 9D, by using a second welding mold 140 constituted by an upper mold 141 and a lower mold 142, peripheral edge parts of the first sheet-shaped member 81 and the second sheet-shaped member 82 thus laminated are welded with each other, and the first sheet-shaped member 81 and the second sheet-shaped member 82 are welded in between the adjacent storage chambers 2 also in regions excluding the region where the cubic figures corresponding to the first communicating part 41 and the second communicating part 42 are formed.

Thereby, the cell cryopreservation container 1 according to the first embodiment is manufactured.

According to the cell cryopreservation container 1 according to the first embodiment described above, there are advantageous effects such as the following.
(1) When a cell cryopreservation container including many storage chambers is stored in the protective case 100, unnecessary space is produced inside the protective case 100 as a result of the existence of the dividing area which divides the storage chambers with each other. Accordingly, the cell cryopreservation container 1 includes the plurality of storage chambers 2 and the dividing area 3 which is arranged between two adjacent storage chambers 2. In addition, the front face part 11 is formed in a convex and concave shape by the plurality of storage chambers 2 and the dividing areas 3, and the back face part 12 is formed planate. Moreover, the length W2 of the dividing area 3 in the width direction WD is longer than the length W1 of the storage chamber 2 in the width direction WD. Thereby, by way of overlapping the two cell cryopreservation containers 1 such that their front face parts 11 oppose each other, the two cell cryopreservation containers 1 can be stored in the protective case 100 in a state where the storage chamber 2 of the first cell cryopreservation container 1 fits in the dividing area 3 of the second cell cryopreservation container 1. Therefore, since the second cell cryopreservation container 1 can be stored using a space (concave-shaped part) formed by the dividing area 3 in a state where the first cell cryopreservation container 1 is stored in the protective case 100, the two cell cryopreservation containers 1 can be stored without having unnecessary space in the protective case 100. In addition, since the back face part 12 of the cell cryopreservation container 1 is formed planate, unnecessary space is not formed between the inner surface of the protective case 100 and the back face part 12 of the cell cryopreservation container 1. As a result, even if the cell cryopreservation container 1 has many storage chambers 2 (especially, three or more), the cells can be efficiently stored in a definite capacity.
(2) The protruding end face 21 in each of the plurality of storage chambers 2 at the side of the front face part 11, and the surface of the dividing area 3 at the side of the front face part 11 are formed planate. Thereby, the protruding end face 21 and the surface of the dividing area 3 at the side of the front face part 11 are brought into close contact with each other when two cell cryopreservation containers 1 are overlapped with each other. Therefore, it is possible to reduce unnecessary space inside the protective case 100 in a state where two cell cryopreservation containers 1 are stored in the protective case 100.
(3) It is configured such that the communicating part 4 includes the first communicating part 41 and the second communicating part 42. Thereby, the cells introduced into the storage chamber 2 from the cell introduction part 6 can be introduced into all of the plurality of storage chambers 2 through the second communicating part 42. In addition, air inside the plurality of storage chambers 2 can be moved through the first communicating part 41 to the storage chamber 2 having the cell introduction part 6, and can be lead out from the cell introduction part 6 to the outside thereafter. Therefore, the cells can be efficiently stored in the plurality of storage chambers 2.
(4) The first communicating part 41 is arranged at the upper end side as compared to the upper end part of the storage chamber 2, and the second communicating part 42 is arranged at the lower end side compared to the lower end side of the storage chamber 2. Thereby, it is possible to prevent interference of the storage chamber 2 with the first communicating part 41 and the second communicating part 42 when the two cell cryopreservation containers 1 are overlapped with each other. Therefore, since the storage chamber 2 and the dividing area 3 are brought into close contact with each other more when two cell cryopreservation containers 1 are overlapped with each other, it is possible to reduce unnecessary space inside the protective case 100 in the state where the two cell cryopreservation containers 1 are stored in the protective case 100. In addition, when two cell cryopreservation containers 1 are overlapped with each other, it is possible to prevent those cell cryopreservation containers 1 from shifting in the height direction HD.
(5) It is configured such that the thickness (length in the thickness direction TD) T2 of the first communicating part 41 and the second communicating part 42 is preferably less than or equal to 1/2 of the thickness (length in the thickness direction TD) T1 of the storage chamber 2, and more preferably less than or equal to 1/4. Thereby, it is possible to reduce interference of the storage chamber 2 with the first communicating part 41 and the second communicating part 42 when two cell cryopreservation containers 1 are overlapped with each other. Therefore, when two cell cryopreservation containers 1 are overlapped with each other, since the storage chamber 2 and the dividing area 3 are brought into close contact with each other, it is possible to reduce unnecessary space inside the protective case 100 in the state where two cell cryopreservation containers 1 are stored in the protective case 100.
(6) The cell introduction part 6 and the plurality of cell withdrawal parts 7 are arranged at the upper end part of the plurality of storage chambers 2. Thereby, in the first welding process S3 in the manufacture of the cell cryopreservation container 1, the cell introduction part 6 and the plurality of cell withdrawal parts 7 can be formed by welding only the upper end side of the laminated first sheet-shaped member 81 and the second sheet-shaped member 82. Therefore, the manufacturing process of the cell cryopreservation container 1 can be simplified.
(7) When collecting the cells stored in the storage chamber 2 from the cell withdrawal part 7, the cells are collected by inserting a syringe needle from the cell withdrawal part 7 into the storage chamber 2. Accordingly, it is configured such that the cell withdrawal part 7 includes a pair of guard parts 73, and the pair of guard parts 73 are arranged at one side and the other side of inside of the storage chamber 2 in the thickness direction TD. Thereby, when collecting the cells stored inside the storage chamber 2 from the cell withdrawal part 7, it is possible to prevent the syringe needle or the like that is inserted for collecting the cells from accidentally piercing the side of the front face part 11 or the side of the back face part 12 of the storage chamber 2. In particular, in the first embodiment, it is possible to prevent the syringe needle from accidentally piercing the back face part 12 which is formed planate and where the tip of the syringe needle may easily contact.

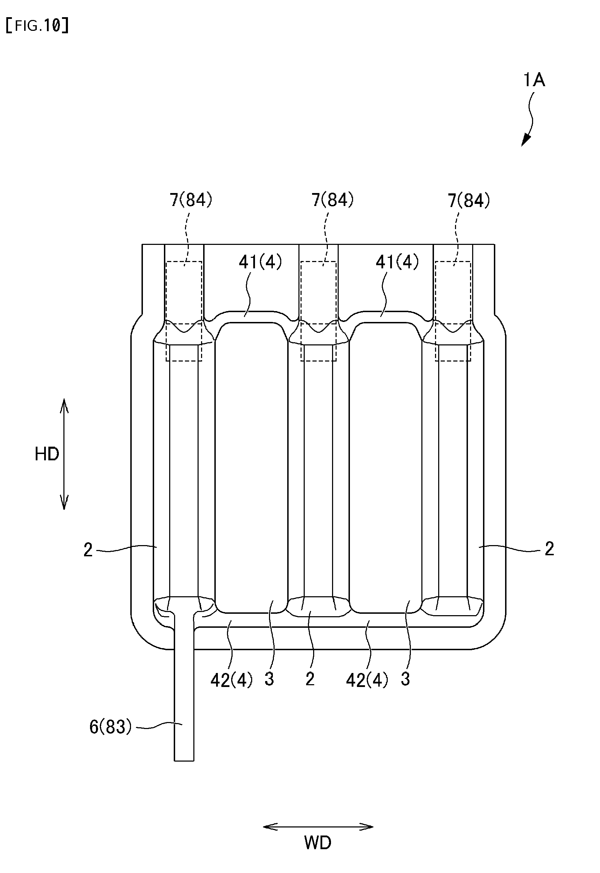

Next, the second embodiment of the cell cryopreservation container of the present invention will be described with reference to FIG. 10. FIG. 10 is a plan view showing the cell cryopreservation container 1A of the second embodiment. It should be noted that, in the descriptions for the second embodiment and later, the same constituting elements are assigned the same reference numerals and their descriptions will be omitted or simplified.

The cell cryopreservation container 1A of the second embodiment is different from the first embodiment in the structure of the cell introduction part 6 and the cell withdrawal part 7.

In the second embodiment, the cell introduction part 6 is arranged at the lower end side of one storage chamber 2 out of the plurality of storage chambers 2. Moreover, the cell withdrawal part 7 is arranged at the upper end part of each of the plurality of storage chambers 2.

According to the cell cryopreservation container 1A of the second embodiment, there are the following advantageous effects in addition to the advantageous effects of (1)-(5) and (7).
(8) The cell withdrawal part 7 is arranged at each of the plurality of storage chambers 2. Thereby, the cell withdrawal part 7 is arranged at the storage chamber 2 having the cell introduction part 6 also, and thus all of the plurality of storage chambers 2 can be isolated from each other. Therefore, the cells stored in the cell cryopreservation container 1 can be used more efficiently.

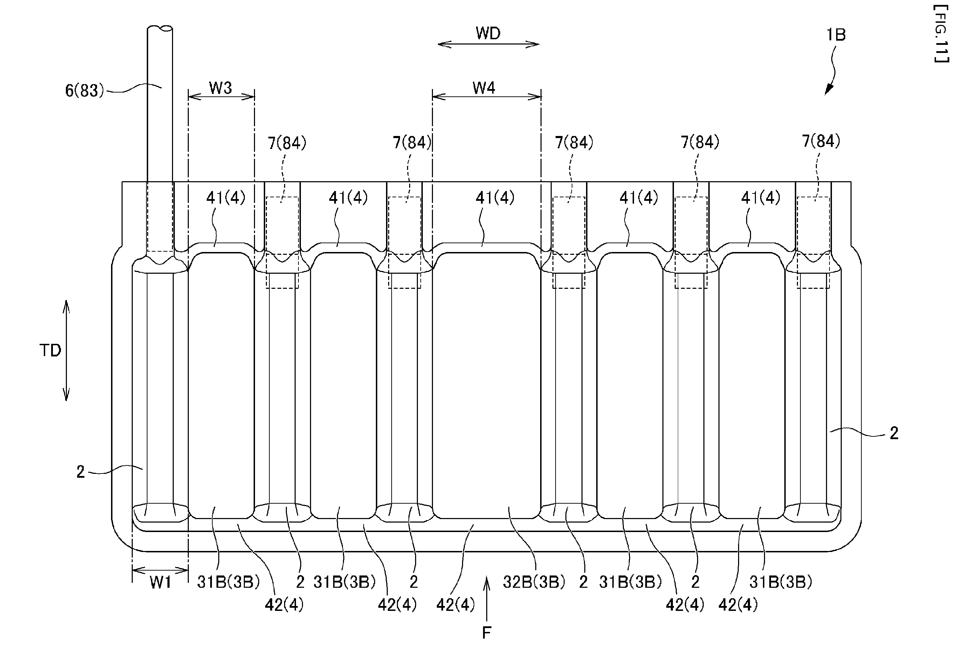

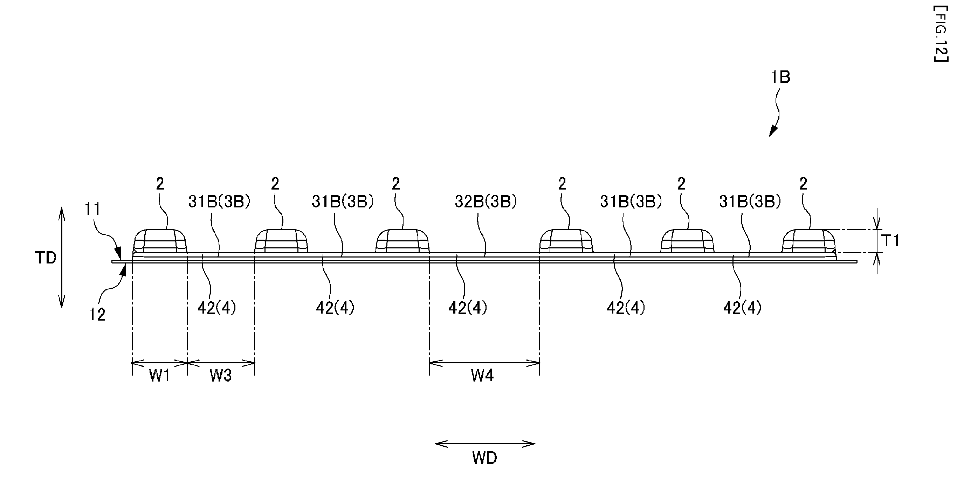

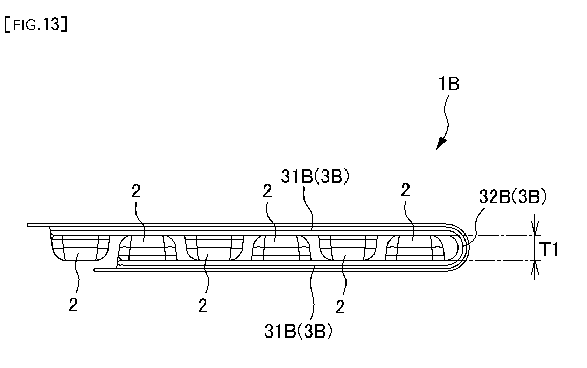

Next, the third embodiment of the cell cryopreservation container according to the present invention will be described with reference to FIG. 11 to FIG. 13. FIG. 11 is a plan view showing the cell cryopreservation container 1B according to the third embodiment and FIG. 12 is a view from Arrow F in FIG. 11. FIG. 13 is a side view showing a state where the cell cryopreservation container 1B according to the third embodiment is folded.

The cell cryopreservation container 1B according to the third embodiment is different from the first embodiment in the number of the storage chambers 2 and the structure of the dividing area 3B.

As shown in FIG. 11 and FIG. 12, the cell cryopreservation container 1B according to the third embodiment includes six storage chambers 2 and five dividing areas 3B. The dividing area 3B according to the third embodiment includes: a first dividing area 31B whose length in the width direction WD is a first length W3; and a second dividing area 32B whose length in the width direction WD is a second length W4, which is longer than the first length W3.

The first dividing area 31B is arranged between storage chambers 2 other than two storage chambers 2 arranged in the center out of the six storage chambers 2. That is, in the third embodiment, the first dividing area 31B is arranged at four locations. First length W3, which is the length of the first dividing area 31B in the width direction WD is preferably 100% to 150% of the length W1 of the storage chamber 2 in the width direction WD.

The second dividing area 32B is arranged between two storage chambers 2 arranged in the center out of the six storage chambers 2. The second length W4, which is the length of the second dividing area 32B in the width direction WD, is 100% to 150% of the sum of the first length W3 and the thickness T1 of the storage chamber 2 from the viewpoint of enabling to fit the storage chamber 2 and the first dividing area 31B with each other suitably when the second dividing area 32B is folded.

As shown in FIG. 13, the cell cryopreservation container 1B according to the third embodiment is stored in the protective case in a state where it is folded in the second dividing area 32B such that the front face parts 11 oppose each other. Here, in the cell cryopreservation container 1B according to the third embodiment, it is configured such that the length W4 of the second dividing area 32B in the width direction WD is longer than the length W3 of the first dividing area 31B. Thereby, the storage chamber 2 and the first dividing area 31B, which oppose each other, can suitably fit when the cell cryopreservation container 1B is folded.

According to the cell cryopreservation container 1B according to the third embodiment, there are the following advantageous effects in addition to the above-described advantageous effects of (2) - (5) and (7).
(9) It is configured such that the dividing area 3B includes the first dividing area 31B where the length in the width direction WD is the first length W3 and the second dividing area 32B where the length in the width direction WD is longer than the first length W3, and the second dividing area 32B is arranged between two storage chambers 2 located in the center of the plurality of storage chambers 2. Thereby, the storage chamber 2 and the first dividing area 31B, which oppose each other, can suitably fit by folding the cell cryopreservation container 1B in the second dividing area 32B such that the front face parts 11 oppose each other. Therefore, since the cell cryopreservation container 1B can be stored without having unnecessary space in the protective case 100, the cells can be efficiently stored in a definite capacity while having a plurality of storage chambers 2.

Although preferred embodiments of the cell cryopreservation container according to the present invention have been described above, the present invention is not limited to the above-described embodiments and may be modified as necessary.

For example, although the cell cryopreservation container 1, 1A includes three storage chambers 2 in the first embodiment and the second embodiment and the cell cryopreservation container 1B includes six storage chambers 2 in the third embodiment, the present invention is not limited to this. That is, the present invention may be configured such that the cell cryopreservation container includes storage chambers of other numbers, such as four, five and eight.

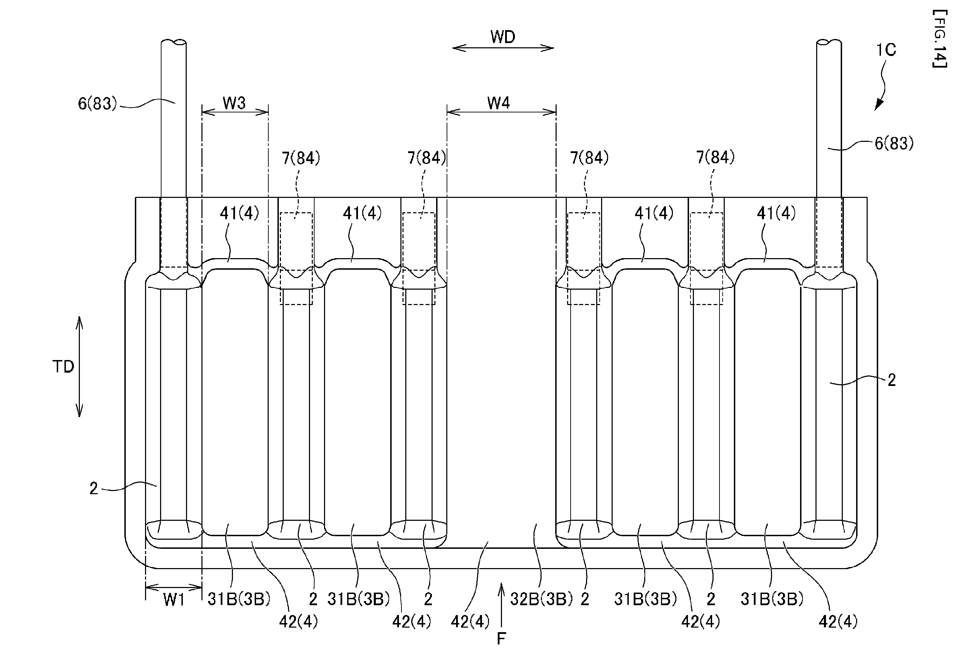

In addition, although the first communicating part 41 and the second communicating part 42 are formed in the first dividing area 31B and the second dividing area 32B in the third embodiment, the present invention is not limited to this. That is, as shown in FIG. 14, it may be configured such that the first communicating part 41 and the second communicating part 42 are formed only in the first dividing area 31B. Thereby, the first communicating part 41 and the second communicating part 42 are not formed in the second dividing area 32B where the folding is carried out when stored in a protective case, and thus the second dividing area 32B can be easily folded.

It should be noted that, in this case, the cell introduction parts 6 may be formed at the storage chambers 2 at both ends out of the six storage chambers 2, respectively, for example.

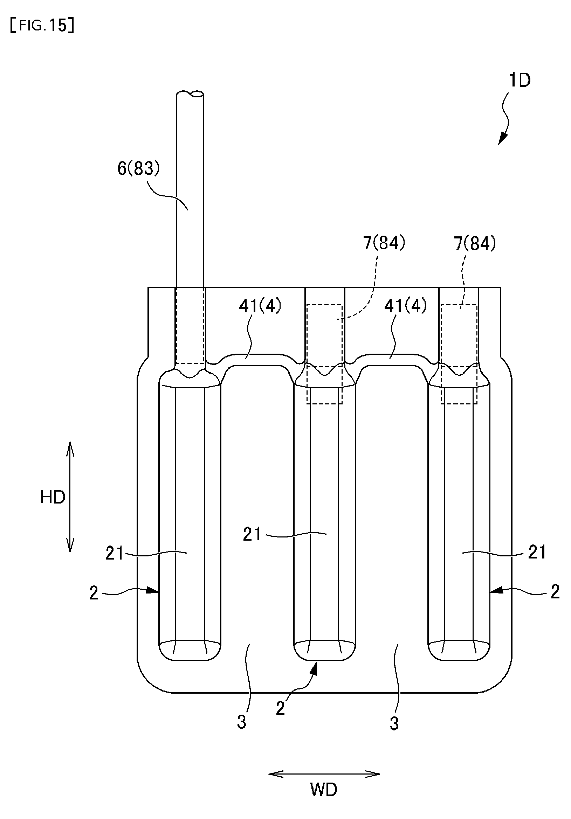

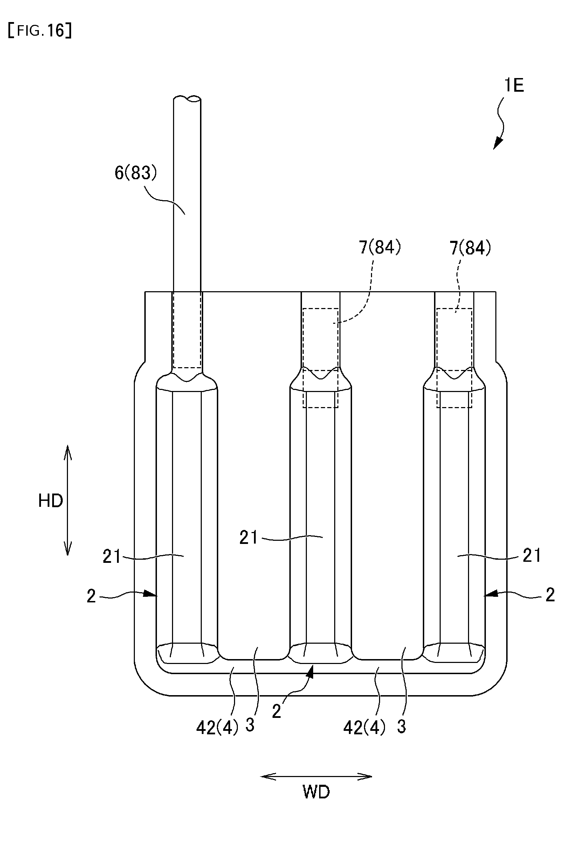

In addition, although the communicating part 4 is configured with the first communicating part 41 and the second communicating part 42 in the first to third embodiments, the present invention is not limited to this. That is, the communicating part 4 may be configured with only the first communicating part 41 as shown in FIG. 15 and the communicating part 4 may be configured with only the second communicating part 42 as shown in FIG. 16.

## Claims

1. A cell cryopreservation container comprising:
a plurality of storage chambers which are arranged at definite intervals in a first direction;
one or a plurality of dividing areas which are arranged between two adjacent storage chambers; and
one or a plurality of communicating parts which are arranged at one end side and/or the other end side in the dividing area in a second direction that is orthogonal to the first direction and connect the two adjacent storage chambers; wherein
a front face part is formed at one side in a third direction that is orthogonal to the first direction and the second direction and a back face part is formed at the other side in the third direction;
the front face part is formed in a convex and concave shape by the plurality of storage chambers and the communicating part protruding to the one side in the third direction and the back face part is formed planate; and
length of the dividing area in the first direction is longer than length of the storage chamber in the first direction.

2. The cell cryopreservation container according to claim 1, wherein
an end surface in each of the plurality of storage chambers at the front face part side and a surface of the dividing area at the front face part side are formed planate.

3. The cell cryopreservation container according to claim 1 or 2, wherein
the communicating part includes:
a first communicating part which is arranged in the dividing area at a side of one end in the second direction; and
a second communicating part which is arranged in the dividing area at a side of the other end in the second direction.

4. The cell cryopreservation container according to claim 3, wherein
the first communicating part is arranged at the one end side in the second direction as compared to a position of the one end part of the storage chamber in the second direction, and
the second communicating part is arranged at the other end side in the second direction as compared to a position of the other end part of the storage chamber in the second direction.

5. The cell cryopreservation container according to any one of claims 1-4, wherein
length of the communicating part in the third direction is less than or equal to 1/2 of length of the storage chamber in the third direction.

6. The cell cryopreservation container according to any one of claims 1-5, further comprising:
a cell introduction part which is provided in one of the plurality of storage chambers and via which a cell is introduced into the plurality of storage chambers; and
a cell withdrawal part which is provided in the plurality of storage chambers and via which the cell stored in the storage chamber is withdrawn.

7. The cell cryopreservation container according to claim 6, wherein
the cell withdrawal part is provided at the one end part of each of the plurality of storage chambers in the second direction, and
the cell introduction part is provided at the other end part of one storage chamber in the second direction out of the plurality of storage chambers.

8. The cell cryopreservation container according to any one of claims 1-7, wherein
the dividing area includes a first dividing area whose length in the first direction is a first length and a second dividing area whose length in the first direction is a second length, which is longer than the first length, and
the second dividing area is arranged between two of the storage chambers located in a center out of the plurality of storage chambers.

9. The cell cryopreservation container according to claim 8, wherein
the communicating part is not arranged in the second dividing area.
